# EUROPEAN PATENT APPLICATION

(11) **EP 4 826 360 A1**
(43) Date of publication of application: **21.10.2026**
(21) Application number: 26172332.4
(22) Date of filing: 14.04.2026
(51) Int. Cl.: G01N 21/64, E04H 4/12, G01N 21/85, G01N 33/18, C02F 103/42, G01N 21/63, G01N 21/84

(54) **SYSTEMS AND METHODS FOR OPTICAL DETECTION OF ALGAE CONCENTRATIONS IN RECREATIONAL BODIES OF WATER**

(30) Priority: 14.04.2025 US 202563788269 P; 13.04.2026 US 202619646097
(71) Applicant: H2flow Controls, Inc., Toledo 43617 (US)
(72) Inventor: HACKETT, Paul, Sylvania, OH (US); HACKETT, Gavin, Sylvania, OH (US); MCDERMOTT, Sean, Perrysburg, OH (US); ILIFF, Andy, Sylvania, OH (US); CSATI, Daniel, Sarnen (CH)
(74) Representative: Manitz Finsterwald Patent- und Rechtsanwaltspartnerschaft mbB

(57) **Abstract**

A system is provided for an algae detector (130, 154, 164, 172, 182). In some examples, the algae detector (130, 154, 164, 172, 182) includes a flow cell (210) disposed within a fluid pathway of a cartridge (212) configured to pass water through the flow cell (210) for algae detection, a light source (216) configured to emit excitation light through the flow cell (210) into the water at a predetermined wavelength selected to cause algae within the water to fluoresce, a photosensor (218) configured to detect an amount of fluorescent light emitted by the algae in response to the excitation light, and an enclosure (208) configured to house the flow cell (210), the cartridge (212), the light source (216), and the photosensor (218). The system is particularly suited to monitor the presence of algae in recreational waters such as swimming pool water.

## Description

### CROSS-REFERENCE TO RELATED APPLICATIONS

This application claims the benefit of U.S. Provisional Application No. 63/788,269, filed on April 14, 2025, the disclosure of which is hereby incorporated by reference in its entirety herein.

### FIELD

The present disclosure relates to detecting algae concentrations in recreational bodies of water and, in particular, to systems and methods for detection of algae concentration in recreational bodies of water using optical lenses and photoelectronic components.

### BACKGROUND

This section provides background information related to the present disclosure which is not necessarily prior art.

Recreational bodies of water, such as residential pools, spas, water features, ponds, etc., are like any other body of water in that they can grow and cultivate algae, which is undesirable for the users of the recreational bodies of water. Current solutions for detecting algae in recreational bodies of water are not commercially feasible in terms of the cost to manufacture and generally utilize a more reactive approach to maintenance in the recreational water industry.

### SUMMARY

One aspect of the present disclosure provides an algae detector. In some examples, the algae detector includes a flow cell disposed within a fluid pathway of a cartridge configured to pass water through the flow cell for algae detection, a light source configured to emit excitation light through the flow cell into the water at a predetermined wavelength selected to cause algae within the water to fluoresce, a photosensor configured to detect an amount of fluorescent light emitted by the algae in response to the excitation light, and an enclosure configured to house the flow cell, the cartridge, the light source, and the photosensor.

One aspect of the present disclosure provides an offline loop algae detection system. In some examples, the system includes a first connector configured to connect to a first portion of a pool recirculation system used to recirculate water for a recreational body of water and a second connector configured to connect to a second portion of the pool recirculation system having a pressure differential differing from that of the first portion, wherein the cartridge of the algae detector includes an inlet port configured to fluidly connect an inlet of the fluid pathway to the first connector and an outlet port configured to fluidly connect an outlet of the fluid pathway to the second connector.

One aspect of the present disclosure provides an algae detection and pool cleaning system. In some examples, the system includes a brush cleaner configured to clean a surface of a swimming pool containing a recreational body of water and a water pump attached to the brush cleaner and configured to pump water from the recreational body through a water pump outlet, wherein the cartridge of the algae detector includes an inlet port configured to fluidly connect an inlet of the fluid pathway to the water pump outlet.

One aspect of the present disclosure provides an algae detection and pool cleaning system. In some examples, the system includes an automatic cleaner configured to automatically clean a surface of a swimming pool containing a recreational body of water using a motorized cleaning element and a water pump attached to the automatic cleaner and configured to pump water from the recreational body through a water pump outlet, wherein the cartridge of the algae detector includes an inlet port configured to fluidly connect an inlet of the fluid pathway to the water pump outlet.

In some examples, the automatic cleaner is one of a robotic cleaner, a suction side cleaner, and a pressure side cleaner.

One aspect of the present disclosure provides an algae detection and pool cleaning system. In some examples, the system includes a self-propelled surface skimmer configured to clean proximate a waterline surface of a recreational body of water and a water pump attached to the self-propelled surface skimmer and configured to pump water from the recreational body through a water pump outlet, wherein the cartridge of the algae detector includes an inlet port configured to fluidly connect an inlet of the fluid pathway to the water pump outlet.

One aspect of the present disclosure provides an algae detection and pool cleaning system. In some examples, the system includes a floating surface skimmer configured to clean proximate a waterline surface of a recreational body of water and a water pump attached to the floating surface skimmer and configured to pump water from the recreational body through a water pump outlet, wherein the cartridge of the algae detector includes an inlet port configured to fluidly connect an inlet of the fluid pathway to the water pump outlet.

One aspect of the present disclosure provides a remote pool monitoring system. In some examples, the system includes a pool monitoring device and the algae detector includes a wireless transceiver configured to wirelessly transmit an algae signal to the pool monitoring device, wherein the algae signal represents the amount of fluorescent light detected by the photosensor.

In some examples, the wireless transceiver is a LoRa transceiver configured to transmit the algae signal over a low-power wide-area network to the pool monitoring device, wherein the pool monitoring device includes a Wi-Fi transceiver configured to transmit pool monitoring data over a Wi-Fi network.

In some examples, the flow cell includes an emitter side facing the light source and a sensor side facing the photosensor, wherein the emitter side is orthogonal to the sensor side.

In some examples, the cartridge includes an inlet port connected proximate to an inlet of the fluid pathway and an outlet port connected proximate to an outlet of the fluid pathway.

In some examples, the enclosure, the cartridge, the inlet port, and the outlet port cooperate to provide a light-tight fit configured to prevent ambient light from reaching the flow cell, the light source, and the photosensor.

In some examples, the inlet port and the outlet port are threadably connected to the cartridge and include raised annular rings configured to secure a flexible hose or tubing.

In some examples, the photosensor is included as part of a sensor assembly having an indicator configured to output an indication of the amount of the fluorescent light detected by the photosensor.

In some examples, the indicator is a visual indicator comprising one or more light emitting diodes visible from an exterior of the enclosure via a light pipe extending from the sensor assembly through a lid of the enclosure.

In some examples, the visual indicator is configured to output a first indication when the amount of algae is below a first algae concentration threshold, a second indication when the amount of algae is between the first algae concentration threshold and a second algae concentration threshold, and a third indication when the amount of algae exceeds the second algae concentration threshold.

One aspect of the present disclosure provides an algae detector. In some examples, the algae detector includes a flow cell assembly including a flow cell disposed within a cartridge, wherein an inner passageway of the flow cell in fluid communication with an inner passageway of the cartridge define a fluid pathway configured to direct water through the cartridge and the flow cell, wherein the cartridge includes an emitter opening to an emitter side of the flow cell and a sensor opening to a sensor side of the flow cell, an emitter assembly including a light source configured to emit excitation light through the emitter opening and the emitter side into the fluid pathway at a predetermined wavelength selected to cause algae in the water to fluoresce, a sensor assembly including a photosensor and a processor, wherein the photosensor is configured to detect fluorescent light emitted through the sensor opening and the sensor side by the algae in response to the excitation light and the processor is configured to generate an algae signal based on an amount of the fluorescent light detected by the photosensor, wherein the algae signal represents an algae concentration in the water, and an enclosure configured to house the flow cell assembly, the emitter assembly, and the sensor assembly.

In some examples, the flow cell includes a body formed from an optically transmissive material and including an emitter side wall configured to permit passage of the excitation light from the light source into the fluid pathway and a sensor side wall configured to permit passage of the fluorescent light emitted by the algae out of the fluid pathway toward the photosensor, wherein the emitter side wall and the sensor side wall are disposed on non-opposing walls of the body and oriented orthogonally to each other.

In some examples, the enclosure includes a lid removably attached to a main body and a seal disposed between the lid and the main body to provide a water-tight fit between the main body and the lid.

One aspect of the present disclosure provides a system for cleaning a swimming pool containing a recreational body of water. In some examples, the system includes a cleaner configured to clean at least one of a surface of the swimming pool and water included in the recreational body of water, a water pump attached to the cleaner and configured to pump water from the recreational body through a water pump outlet, a remote pool monitoring device including a wireless pool transceiver configured to transmit pool monitoring data over a wireless network, and an algae detector including a flow cell disposed within a fluid pathway of a cartridge configured to pass water from the recreational body of water through the flow cell for algae detection, a light source configured to emit excitation light through the flow cell into the fluid pathway at a predetermined wavelength selected to cause algae within the water to fluoresce, a photosensor configured to detect an amount of fluorescent light emitted by the algae in response to the excitation light, and a wireless detector transceiver configured to transmit data representative of the amount of fluorescent light to the wireless pool transceiver.

Further areas of applicability of the present disclosure will become apparent from the detailed description, the claims, and the drawings. The detailed description and specific examples are intended for purposes of illustration only and are not intended to limit the scope of the disclosure.

### BRIEF DESCRIPTION OF THE DRAWINGS

The present disclosure will become more fully understood from the detailed description and the accompanying drawings, wherein:
FIG. 1 is a functional block diagram representing an algae detection and pool cleaning system in accordance with an example of the present disclosure;
FIG. 2 is a perspective view representing an algae detector in accordance with an example of the present disclosure;
FIG. 3 is a cross-sectional view taken along line 3-3 of FIG. 2 in accordance with an example of the present disclosure;
FIG. 4 is a perspective view representing an enclosure of the algae detector shown in FIG. 2 in accordance with an example of the present disclosure;
FIG. 5 is a cross-sectional view taken along line 5-5 of FIG. 4 in accordance with an example of the present disclosure;
FIG. 6 is a perspective view representing a cartridge of the algae detector shown in FIG. 2 in accordance with an example of the present disclosure;
FIG. 7 is a cross-sectional view taken along line 7-7 of FIG. 6 in accordance with an example of the present disclosure;
FIG. 8 is a partial assembly view of the algae detector shown in FIG. 2 in accordance with an example of the present disclosure;
FIG. 9 is a perspective view representing an optical stack housing of the algae detector shown in FIG. 2 in accordance with an example of the present disclosure;
FIG. 10 is a cross-sectional view taken along line 10-10 of FIG. 9 in accordance with an example of the present disclosure; and
FIG. 11 is a flowchart of a method for detecting algae concentrations in accordance with an example of the present disclosure.

In the drawings, reference numbers may be reused to identify similar and/or identical elements.

### DETAILED DESCRIPTION

Example embodiments will now be described more fully with reference to the accompanying drawings.

The present disclosure is directed to systems and methods for detecting algae concentrations in recreational bodies of water using optical lenses and photoelectronic components. Specifically, a light-emitting diode (LED) with a specific wavelength (determined by the fluorescent properties of cyanobacteria) emits light through a lens that channels the light into a flow cell (with specific dimensions determined through optical simulation) where water will flow through and any cyanobacteria within the flow cell will fluoresce. The fluorescent light is emitted by the cyanobacteria and passes through an optical filter and another lens that channels the light into a photosensor, such as a photodiode. The photodiode receives the fluorescent light and sends a signal to accompanying signal processing electronics, such as a processor and memory configured to perform the described functionality, that process the signal into one of a few types of signals that can be used to communicate with outside devices to inform the user of the status of the body of water.

The sensor portion is housed in an enclosure that blocks out ambient light (wavelengths from ~10 nm to ~740 nm). This is because light from outside the enclosure will interfere with the wavelength of light being emitted by the LED and thus provide false readings from the photodiode to the processing electronics. The enclosure, for example, can be made of acrylonitrile butadiene styrene (ABS) plastic with a wall thickness of at least 2 mm, although other sufficient wall thicknesses can be used.

The systems of the present disclosure can output the following types of signals: 4-20 mA, RS485, Cellular, serial (SPI, I2C, UART), Wi-Fi, or Bluetooth (standard or BLE). These signals can be used to communicate to an external mobile application on a smartphone, such as iOS and Android devices, as inputs to an automation system or utilized as an input to auto floor cleaning devices. As the system includes wireless connectivity communication functionality, the system can periodically test its connectivity and notify the user of any issues.

The deployment methods or embodiments of the present disclosure include: in-line (plumbed into the return line of the recreational body of water's plumbing system), a floating device (integrated into a floating housing that travels throughout the recreational body of water), and/or integrated into an auto floor cleaning device.

The systems and methods of the present disclosure include a feature that makes the flow cell easily cleanable to increase the longevity of the flow cell and maintain the reliability of the system's ability to accurately monitor the recreational body of water for cyanobacteria.

The logic used by the system to monitor the recreational body of water for cyanobacteria and appropriately alert or notify the user of the status is accomplished by averaging signals (acquired by sampling the flow of water through the flow cell) over a defined period of time. The sample rate and levels at which to alert the user are determined first by the type of environment in which the system is installed and secondly by the deployment method. Both the environment and deployment method of the system can be set either by the user at the time of installation or by the manufacturer at the time of manufacture.

In some examples, the status indicator of the system is based on a "traffic light" type status hierarchy: Go, Caution, Stop. "Go" status tells the user that their recreational body of water is operating at the desirable level. "Caution" status tells the user that they should take preemptive action against further cultivation of cyanobacteria growth in the water. "Stop" status tells the user that the water is at an undesirable level and they should take action to remediate the cyanobacteria growth. This status indication is determined within the logic by which the system is operating based on the environment and deployment method set either by the user at the time of installation or the manufacturer at the time of manufacture.

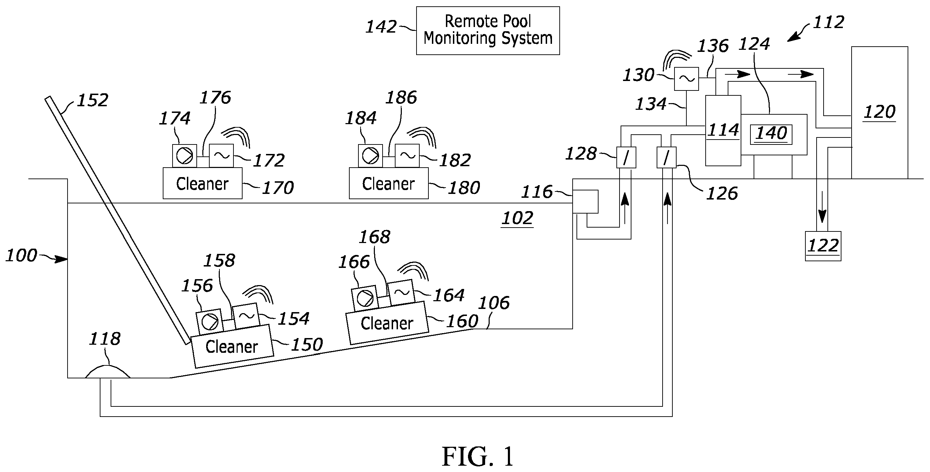

Referring to FIG. 1, a system for detecting algae concentrations within a swimming pool 100 containing a recreational body of water 102 is observable in accordance with one aspect of the present disclosure. The system is shown for exemplary purposes with respect to a variety of algae detectors cooperating with a variety of cleaners to facilitate detecting algae concentrations within the water comprising the recreational body of water 102 and/or doing so while concurrently cleaning a surface 106 of the swimming pool 100 and/or portions of the recreational body of water 102. While the system is illustrated with multiple algae detectors and multiple cleaners, the present disclosure is not necessarily so limited and fully contemplates using more or fewer algae detectors and/or more or fewer cleaners.

The system is shown to include a pool recirculation system 112 configured to clean and filter the recreational body of water 102. The pool recirculation system 112, for example, includes a pump 114 configured to recirculate water from the swimming pool 100 through a skimmer 116 and a main drain 118 for cleaning and filtering with a filter 120 prior to recirculating back the cleaned and filtered water to the swimming pool 100 via a return 122. In particular, the pump 114 is configured to pump water from the swimming pool 100 through the pool recirculation system 112, and the filter 120 is configured to remove contaminants, particulates, and other impurities from the water. The pool recirculation system 112 includes a control module 124 configured to selectively control the opening and closing of valves 126, 128 and the operation of the pump 114 and the filter 120 according to a wide range of control methodologies.

The pool recirculation system 112 and the attendant connections between the skimmer 116 and the main drain 118 are presented functionally and without limitation to represent a variety of features operable in accordance with the present disclosure to facilitate recirculating water from the swimming pool 100 for purposes of cleaning and filtering. The cleaning and filtering are also shown functionally and without limitation to be performed using the pump 114 and the filter 120. The present disclosure is equally applicable to other arrangements utilized to recirculate or otherwise obtain dirty water from the swimming pool 100 and thereafter return clean water, including arrangements that include additional pumps and filters and/or other components to assist in cleaning and filtering water or other fluids included in the swimming pool 100.

In one example, an offline loop algae detection arrangement of the system comprises an algae detector 130 connected across the pump 114. The algae detector 130, for example, is configured to detect an algae concentration within the water being recirculated through the pool recirculation system 112. The offline loop algae detection system, for instance, includes a first connector 134 configured to connect a first portion of the pool recirculation system 112 to an inlet of the algae detector 130 and a second connector 136 configured to connect an outlet of the algae detector 130 to a second portion of the pool recirculation system 112. With this connection across the pump 114, the inlet and the outlet of the algae detector 130 experience a pressure differential from differing water pressures at an inlet and an outlet of the pump 114 that causes a portion of the water being recirculated to flow through the algae detector 130.

The illustrated connection of the algae detector 130 to the pool recirculation system 112 advantageously enables the algae detector 130 to be added to the pool recirculation system 112 as an aftermarket component, i.e., as a component added after installation of the pool recirculation system 112. This advantageous aftermarket installation of the algae detector 130 is presented without limitation as the present disclosure fully contemplates the algae detector 130 being installed concurrently with construction of the pool recirculation system 112. Additionally, the algae detector 130 is shown to be installed offline, i.e., outside of the piping used to connect the water being recirculated to the pump 114, without limitation. The present disclosure also fully contemplates the algae detector 130 being connected inline relative to the pool recirculation system 112, i.e., within the piping used to connect the water being recirculated to the pump 114.

In operation, the algae detector 130 is configured to determine an algae concentration within the water being recirculated through the pool recirculation system 112 and to report the algae concentration or a signal representative thereof to a pool monitoring device 140. The pool monitoring device 140, for example, is included as part of the control module 124 and configured to facilitate communicating data received from the algae detector 130 to a remote pool monitoring system 142. The remote pool monitoring system 142, for example, includes user interfaces, processing tools, controls, and other features associated with controlling and monitoring processes associated with the control module 124. In some examples, the remote pool monitoring system 142 is configured to interact with a mobile device, a control panel, or other device or interface associated with an owner or other entity tasked with cleaning or monitoring the swimming pool 100.

The remote pool monitoring system 142, for instance, is utilized as a humanmachine interface (HMI) through which a pool owner is able to monitor algae concentration levels within the recreational body of water 102 and receive alerts when algae concentrations exceed predetermined thresholds. In one example, the remote pool monitoring system 142 is configured to display real-time algae concentration data received from the algae detector 130 and to generate notifications alerting the pool owner or pool service technician when the algae concentration exceeds a first threshold indicative of a caution condition or a second threshold indicative of a stop condition requiring immediate remedial action such as chemical treatment of the recreational body of water 102.

The remote pool monitoring system 142 may further be configured to communicate with the control module 124 to automatically adjust operation of the pump 114, the filter 120, or the valves 126, 128 in response to detected algae concentration levels, for example by increasing recirculation flow rate or activating a chemical dosing system to introduce algaecide or chlorine into the recreational body of water 102. In some examples, the remote pool monitoring system 142 is further configured to maintain a historical log of algae concentration measurements received from the algae detector 130 to enable trend analysis and predictive maintenance scheduling for the swimming pool 100. The remote pool monitoring system 142, for instance, is configured to enable a pool owner to set a cleaning schedule, e.g., a schedule by which an entirety of the recreational body of water 102 is to be cycled through the pool recirculation system 112.

In one example, an algae detection and pool cleaning arrangement of the system includes a brush cleaner 150 configured to clean the surface 106 of the swimming pool 100. The brush cleaner 150, for example, includes bristles or another cleaning implement configured to be manually moved relative to the surface 106 via an elongated pole 152 or other connection mechanism for purposes of cleaning the surface 106 and/or the nearby water. An algae detector 154 is connected to the brush cleaner 150, or optionally the pole 152, to facilitate detecting algae concentrations in the nearby water. A water pump 156 is attached to the brush cleaner 150 and configured to pump the nearby water through a water pump outlet 158 to an inlet of the algae detector 154. The water pump 156 is shown to be separate from the algae detector 154 without limitation as the water pump 156 may be included as part of the algae detector 154.

In a similar algae detection and pool cleaning arrangement, the system includes an automatic cleaner 160 configured to automatically clean the surface 106 of the swimming pool 100 using a motorized cleaning element. The automatic cleaner 160, for example, includes one of a robotic cleaner, a suction side cleaner, and a pressure side cleaner, each configured to be automatically moved relative to the surface 106 for purposes of cleaning the surface 106 and/or the nearby water without requiring manual operation by a user. An algae detector 164 is connected to the automatic cleaner 160 to facilitate detecting algae concentrations in the nearby water. A water pump 166 is attached to the automatic cleaner 160 and configured to pump the nearby water through a water pump outlet 168 to an inlet of the algae detector 164.

In another algae detection and pool cleaning arrangement, the system includes a self-propelled surface skimmer 170 configured to clean proximate a waterline surface of the recreational body of water 102. The self-propelled surface skimmer 170, for example, includes a motorized drive mechanism configured to propel the self-propelled surface skimmer 170 across the waterline surface of the recreational body of water 102 for purposes of removing debris and contaminants from the waterline surface and/or the nearby water. An algae detector 172 is connected to the self-propelled surface skimmer 170 to facilitate detecting algae concentrations in the nearby water. A water pump 174 is attached to the self-propelled surface skimmer 170 and configured to pump the nearby water through a water pump outlet 176 to an inlet of the algae detector 172.

In yet another algae detection and pool cleaning arrangement, the system includes a floating surface skimmer 180 configured to clean proximate a waterline surface of the recreational body of water 102. The floating surface skimmer 180, for example, includes a buoyant body configured to float on the waterline surface of the recreational body of water 102 and a cleaning implement configured to remove debris and contaminants from the waterline surface and/or the nearby water without requiring self-propulsion. An algae detector 182 is connected to the floating surface skimmer 180 to facilitate detecting algae concentrations in the nearby water. A water pump 184 is attached to the floating surface skimmer 180 and configured to pump the nearby water through a water pump outlet 186 to an inlet of the algae detector 182.

The algae detectors 130, 154, 164, 172, 182 optionally include a wireless transceiver (shown with wireless signal symbols) configured to wirelessly transmit an algae signal or other data to the pool monitoring device 140. The algae signal, for example, represents the amount of fluorescent light detected by a photosensor of the algae detector 130 and is used by the pool monitoring device 140 and/or the remote pool monitoring system 142 to determine the algae concentration in the water. The wireless transceiver, for example, is configured to transmit the algae signal to the remote pool monitoring system 142 in real time or at predetermined intervals, for example at intervals of one second, one minute, or another interval selected based on the application requirements and the desired frequency of algae concentration monitoring.

In one example, the wireless transceiver is configured to transmit the algae signal as a digital data packet representative of the amount of fluorescent light detected by the photosensor. The digital data packet, for example, includes a value representative of the algae concentration in the water, a timestamp associated with the measurement, and an identifier associated with the algae detector 130 to enable the remote pool monitoring system 142 to identify the source of the algae signal when multiple algae detectors are deployed within the system. The wireless transceiver may additionally be configured to receive control signals from the remote pool monitoring system 142, for example to adjust operating parameters of the algae detector 130 such as measurement frequency, detection thresholds, or indicator output settings.

In one example, the wireless transceiver is a LoRa transceiver configured to transmit the algae signal over a low-power wide-area network to the remote pool monitoring system 142. LoRa, which stands for Long Range, is a spread-spectrum modulation technique derived from chirp spread spectrum technology that enables long-range wireless communication with low power consumption. The LoRa transceiver, for example, is configured to transmit the algae signal at a frequency selected from one of the 915 MHz, 868 MHz, or 433 MHz frequency bands depending on the regulatory requirements of the jurisdiction in which the system is deployed. The use of a LoRa transceiver in the algae detector 130 advantageously enables wireless communication between the algae detector 130 and the remote pool monitoring system 142 through the recreational body of water 102.

Similarly, the pool monitoring device 140 includes a transceiver configured to transmit pool monitoring data over a wireless or wired network to the remote pool monitoring system 142, e.g., a Wi-Fi transceiver. The remote pool monitoring system 142, for example, is configured to receive the algae signal from the LoRa transceiver of the algae detector 130 via the low-power wide-area network and to process the algae signal to determine the algae concentration in the water. The pool monitoring device 140 is further configured to transmit pool monitoring data representative of the algae concentration and other monitored parameters to a mobile device, a control panel, or other device or interface associated with an owner or other entity tasked with cleaning or monitoring the swimming pool 100 via the Wi-Fi transceiver over a Wi-Fi network.

The combination of the LoRa transceiver and the Wi-Fi transceiver in the remote pool monitoring system 142 advantageously provides a two-stage wireless communication architecture in which the low-power long-range LoRa link is used to transmit the algae signal from the algae detectors 130, 154, 164, 172, 182 to the remote pool monitoring system 142 and the Wi-Fi link is used to communicate pool monitoring data from the pool monitoring device 140 to user devices and cloud-based services. The present disclosure is not limited to LoRa and Wi-Fi as the wireless communication technologies. Other wireless communication technologies are contemplated and are fully within the scope of the present disclosure, including without limitation Bluetooth, Zigbee, Z-Wave, cellular including 4G LTE and 5G, satellite, and other low-power wide-area network technologies including Sigfox and NB-loT.

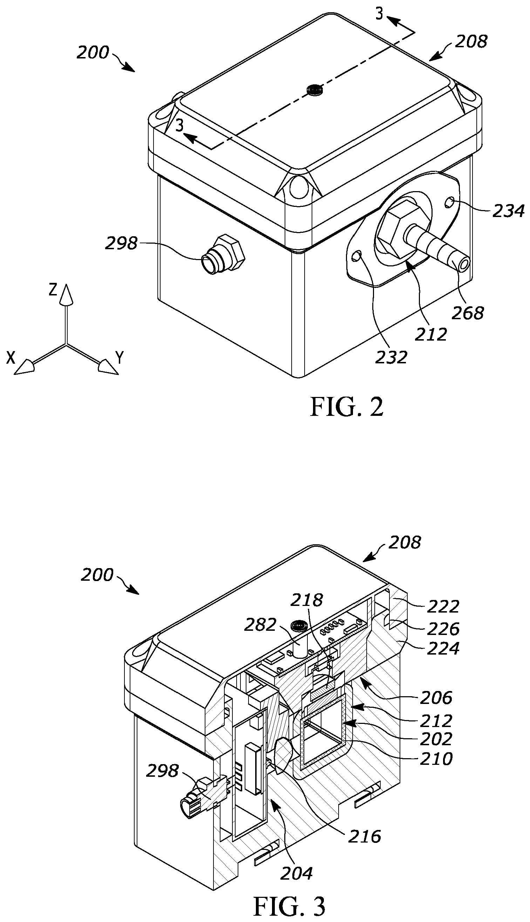

Referring to FIGS. 2-3, FIG. 2 illustrates a perspective view of an algae detector 200, and FIG. 3 illustrates a cross-sectional view of the algae detector 200 taken along line 3-3 of FIG. 2 in accordance with one aspect of the present disclosure. The algae detector 200 is representative of an exemplary configuration of components that may be employed to form the algae detectors 130, 154, 164, 172, 182 described above with respect to FIG. 1, understanding that the various arrangements described with respect to FIG. 1 are not limited to the algae detector 200 shown in FIGS. 2-3. In other words, the algae detector 200 shown in FIG. 2 is representative of a wide range of devices suitable for use as one of the algae detectors 130, 154, 164, 172, 182 shown in FIG. 1.

The algae detector 200 includes a flow cell assembly 202, an emitter assembly 204, a sensor assembly 206, and an enclosure 208 that cooperate to detect an amount of algae or an algae concentration, if any, of a water sample taken from the recreational body of water 102 or other water source for which algae detection is desired. The algae in the water sample or in other identifiable portions of the swimming pool 100 is determined based on data provided by or collected with the algae detector 200 and quantified according to an amount of algae, an algae concentration, or other algae metric. For the sake of presentation simplicity, and without limitation, the algae detected in the water sample is interchangeably referred to as an algae concentration.

In operation, the algae detector 200 is configured to operate by itself or in communication with the pool monitoring device 140 and/or the remote pool monitoring system 142 to determine the algae concentration based on subjecting the water sample to an excitation light having a predetermined wavelength selected to cause algae in the water to fluoresce and detecting an amount of fluorescent light emitted by algae in the water sample in response to the excitation light. As one skilled in the art will appreciate, fluorescence is a photophysical phenomenon in which a substance absorbs electromagnetic radiation at a specific excitation wavelength and subsequently re-emits light in response. The difference between the excitation wavelength of the excitation light and the emission wavelength of the fluorescent light emitted in response is known as the Stokes shift and is a characteristic property of a given fluorescent substance.

As will be more fully appreciated in view of the description below, the algae detector 200 can be similarly configured to facilitate detecting other substances within the recreational body of water 102, such as but not limited to metals that may stain or otherwise discolor the swimming pool 100 and/or the recreational body of water 102. The present disclosure is predominantly described with respect to the algae detector 200 mainly detecting algae due to a long-established need to reliably and efficiently utilize the technology within recreational bodies of water. With respect to algae detection, chlorophyll and phycocyanin are photosynthetic pigments naturally present in green algae and cyanobacteria and are well-characterized naturally fluorescent molecules. These pigments have predictable and well-documented excitation and emission spectra. For example, chlorophyll-a is commonly excited in the blue region of the visible spectrum, at approximately 430 to 450 nanometers, and emits fluorescent light in the red region of the visible spectrum, at approximately 680 to 700 nanometers.

By selecting an excitation wavelength for the excitation light that matches an absorption peak of a target photosynthetic pigment, i.e., a type of algae, and by pairing that excitation light with a spectral filter matched to the emission wavelength of that pigment, the algae detector 200 is configured to selectively detect fluorescent emissions characteristic of green algae and cyanobacteria. The intensity of the fluorescent emission detected by the algae detector 200 is generally proportional to the concentration of the fluorescent substance present within the water sample, thereby enabling the system to quantify the algae concentration present in the recreational body of water 102. Accordingly, the algae detector 200 is described with respect to generating excitation light at a predetermined wavelength, understanding that the wavelength need not be fixed, may be varied during operation, and may otherwise be altered to facilitate detecting different types of algae and/or other substances.

In one example, the flow cell assembly 202 includes a flow cell 210 disposed within a cartridge 212 configured to pass water through the flow cell 210, the emitter assembly 204 includes a light source 216 configured to emit the excitation light, and the sensor assembly 206 includes a photosensor 218 configured to detect fluorescent light emitted in the water sample in response to the excitation light, for algae detection. The enclosure 208 includes a lid 222 removably attached to a main body 224, optionally with a seal 226 disposed between the lid 222 and the main body 224 to provide a water-tight fit operable to waterproof the emitter assembly 204 and the sensor assembly 206 when submerged in the recreational body of water 102.

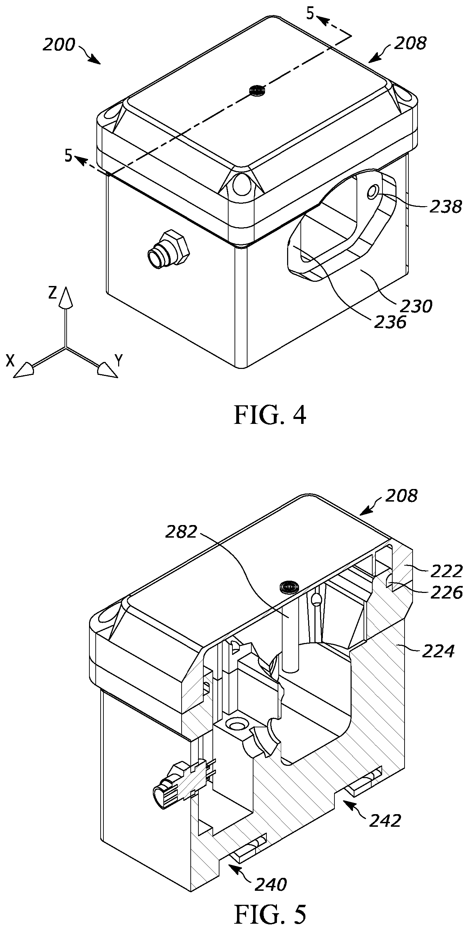

Referring to FIGS. 4-5, FIG. 4 illustrates a perspective view of the enclosure 208 while the cartridge 212 is removed, and FIG. 5 illustrates a cross-sectional view of the enclosure 208 taken along line 5-5 of FIG. 4 in accordance with one aspect of the present disclosure. As shown, the main body 224 includes a through-hole opening 230 extending through the enclosure 208 from one side to the other that is configured to removably retain the cartridge 212, such as with removable fasteners 232, 234 (see FIG. 3) retained within fastener retainers 236, 238 or through other retention mechanisms. The removability of the cartridge 212 is advantageous in accessing the cartridge 212 for cleaning and/or replacement. As also shown, the main body 224 optionally includes keyhole slots 240, 242 for mounting.

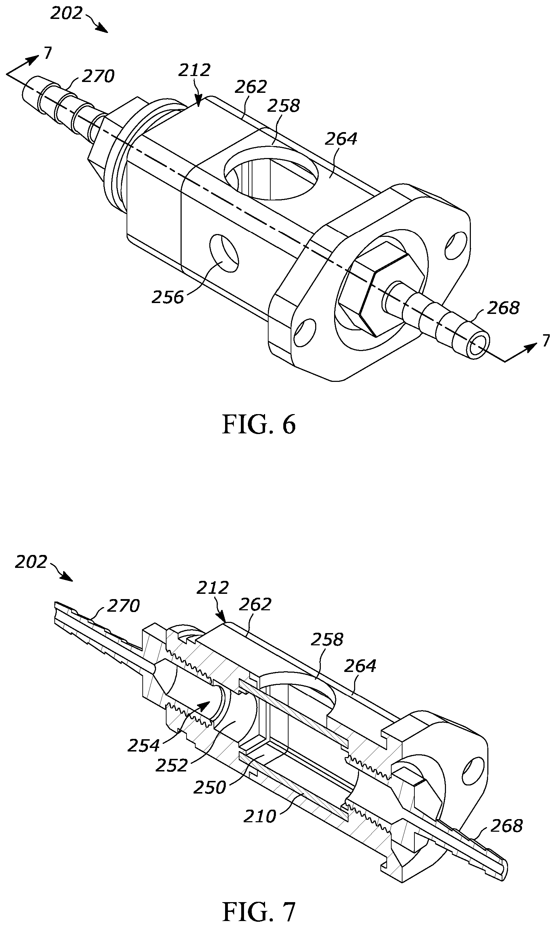

Referring to FIGS. 6-7, FIG. 6 illustrates a perspective view of the flow cell assembly 202, and FIG. 7 illustrates a cross-sectional view of the flow cell assembly 202 taken along line 7-7 of FIG. 6 in accordance with one aspect of the present disclosure. As shown, the cartridge 212 retains the flow cell 210 such that an inner passageway 250 of the flow cell 210 in fluid communication with an inner passageway 252 of the cartridge 212 define a fluid pathway 254 configured to direct sampled water through the cartridge 212 and the flow cell 210. In the illustrated example, the cartridge 212 includes an emitter opening 256 to an emitter side of the flow cell 210 and a sensor opening 258 to a sensor side of the flow cell 210, an end cap 262 attached to a mounting flange 264, such as with glue or a mechanical feature, an inlet port 268 connected proximate an inlet to the fluid pathway 254, and an outlet port 270 connected to an outlet of the fluid pathway 254.

The inlet port 268 and the outlet port 270 are shown for exemplary purposes to be threadably connected to the end cap 262 and the mounting flange 264. The inlet port 268 and the outlet port 270 are also shown for exemplary purposes to include raised annular rings configured to secure the cartridge 212 relative to flexible hosing, tubing, and other connecting elements that may be incorporated to facilitate directing water relative to the cartridge 212. With respect to the arrangement shown in FIG. 1, for example, the inlet port 268 is configured to connect to the first connector 134 or the water pump outlets 158, 168, 176, 186, and the outlet port 270 is configured to connect to the second connector 136 or to output received water back to the recreational body of water 102.

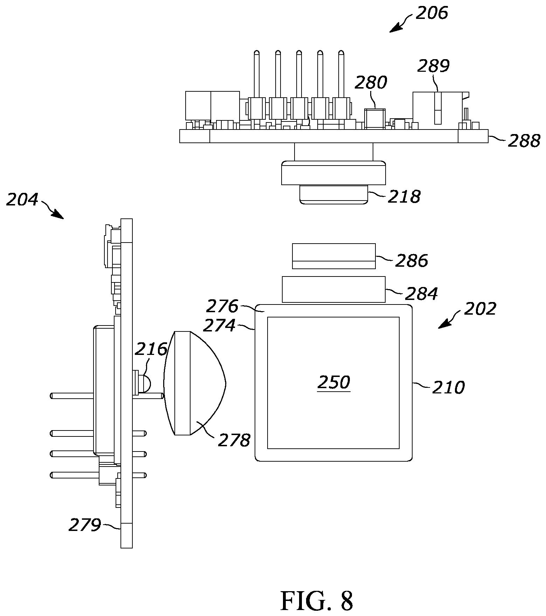

Turning to FIG. 8, a partial assembly view is presented to functionally illustrate relative positioning of the flow cell assembly 202 to the emitter assembly 204 and the sensor assembly 206. As shown, the flow cell assembly 202 includes an emitter side facing the light source 216 and a sensor side facing the photosensor 218. The flow cell 210 includes a body formed from an optically transmissive material and including an emitter side wall 274 configured to permit passage of the excitation light from the light source 216 into the fluid pathway 254 and a sensor side wall 276 configured to permit passage of the fluorescent light emitted by the algae out of the fluid pathway toward the photosensor 218. In this exemplary configuration, the emitter side wall 274 and the sensor side wall 276 are disposed on non-opposing walls of the body and oriented orthogonally to each other.

The orthogonal orientation of the light source 216 relative to the photosensor 218 is beneficial in limiting the excitation light from traveling directly toward the photosensor 218. In fluorescence-based detection systems of the type described herein, the excitation light emitted by the light source 216 is orders of magnitude more intense than the fluorescent light emitted by the algae in response to the excitation light. If the light source 216 and the photosensor 218 were arranged in a diametrically opposed configuration, the transmitted excitation light would travel directly toward the photosensor 218 and could overwhelm the relatively weak fluorescent signal even in the presence of an optical filter disposed between the flow cell 210 and the photosensor 218.

By orienting the light source 216 and the photosensor 218 orthogonally to each other, the direct optical path of the excitation light does not intersect the photosensor 218, such that the photosensor 218 receives primarily fluorescent light emitted laterally from the algae within the fluid pathway 254 in response to the excitation light. Optionally, the enclosure 208 and the cartridge 212 cooperate to define a light-tight housing configured to prevent ambient light from entering the enclosure 208 and reaching the flow cell 210, the emitter assembly 204, and the sensor assembly 206. By way of example, the optically opaque material forming the enclosure 208 and the cartridge 212, for example, is a black acrylonitrile butadiene styrene material having a light transmission of less than 0.1 percent per surface that absorbs substantially all ambient light incident on the exterior surfaces of the enclosure 208 and prevents the ambient light from reaching the interior of the enclosure 208.

In some examples, the emitter assembly 204 includes a conical lens 278 directing light from the light source 216 and an emitter printed circuit board (PCB) 279 configured to support and electrically connect the light source 216 and associated drive circuitry configured to control operation of the light source 216. The conical lens 278, for example, is an aspheric condenser lens configured to collimate the excitation light into a focused beam. The emitter PCB 279, for example, includes a current driver configured to supply a regulated drive current to the light source 216 at a level selected to cause the light source 216 to emit excitation light at the predetermined wavelength and at a predetermined intensity selected to produce a detectable fluorescent response from algae present in the water flowing through the fluid pathway 254. The emitter PCB 279 may further include thermal management components configured to dissipate heat generated by the light source 216.

In some examples, the sensor assembly 206 includes an indicator 280 configured to output an indication of the amount of the fluorescent light detected by the photosensor 218. The indicator 280, for example, is a visual indicator comprising one or more light emitting diodes visible from an exterior of the enclosure via a light pipe 282 (see FIG. 3) extending from the sensor assembly 206 through the lid 222. By way of example, the visual indicator 280 is configured to output a first indication, e.g., a green light, when the amount of algae is below a first algae concentration threshold, a second indication, e.g., a yellow light, when the amount of algae is between the first algae concentration threshold and a second algae concentration threshold, and a third indication, e.g., a red light, when the amount of algae exceeds the second algae concentration threshold

In some examples, the sensor assembly 206 includes a spectral filter 284 and a cylindrical lens 286, where the spectral filter 284 is configured to transmit fluorescence emission wavelengths while blocking the predetermined excitation wavelength of the excitation light emitted by the light source 216 to prevent excitation light from reaching the photosensor 218, and the cylindrical lens 286 is configured to collect and focus the fluorescent light passing through the spectral filter 284 onto the active area of the photosensor 218 to increase the efficiency of fluorescent light collection and improve the sensitivity of the algae detector 200. The spectral filter 284 and the cylindrical lens 286 are disposed between the sensor side wall 276 of the flow cell 210 and the photosensor 218 in the optical path of the fluorescent light traveling from the fluid pathway 254 toward the photosensor 218.

In some examples, the sensor assembly 206 includes a sensor PCB 288 including a variety of electronic components, including a processor 289 configured to generate the algae signal and/or the wireless transceiver described above. The sensor PCB 288 brings together the core electronic functions of the algae detector 200, addressing processing needs associated with generating the algae signal based on the fluorescent light detected by the photosensor 218 and relatedly communicating the algae signal or other data to the pool monitoring device 140. Of course, the sensor assembly 206 may include a variety of other components, in addition to or in place of those described herein.

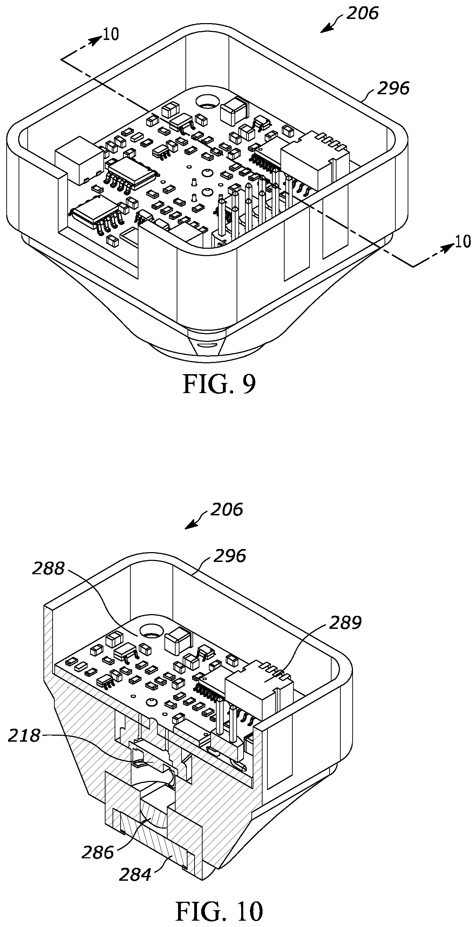

Referring to FIGS. 9-10, FIG. 9 illustrates a perspective view of an optical stack housing 296 for the sensor assembly 206, and FIG. 10 illustrates a cross-sectional view of the optical stack housing 296 taken along line 10-10 of FIG. 9 in accordance with one aspect of the present disclosure. As shown, the optical stack housing 296 is configured to retain the sensor PCB 288 relative to the photosensor 218, the cylindrical lens 286, and the spectral filter 284. Optionally, the emitter PCB 279 and the sensor PCB 288 are powered via an electrical connection 298 (see FIG. 2). The electrical connection 298 is shown for exemplary purposes to correspond with a threaded direct current (DC) power connector configured to directly power the emitter PCB 279 and the sensor PCB 288 and/or to charge batteries included therein.

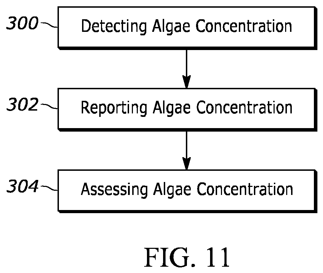

Turning to FIG. 11, a flowchart of a method for detecting algae concentrations in a recreational body of water using the algae detector 200 is observable in accordance with one aspect of the present disclosure. At 300, the method includes detecting algae concentrations based on directing a water flow through a fluid pathway of a flow cell disposed within a cartridge, emitting excitation light from a light source through the flow cell into the water flow at a predetermined wavelength selected to cause fluorescence of algae in the water flow, detecting an amount of fluorescent light emitted by the algae in response to the excitation light using a photosensor disposed orthogonally to the light source relative to the flow cell, generating an algae signal based on the amount of fluorescent light detected by the photosensor, and outputting an indication of the algae concentration based on the algae signal.

At 302, the method includes a reporting process for wirelessly transmitting the algae signal from a wireless transceiver of the algae detector to a remote pool monitoring device, processing the algae signal at the remote pool monitoring device to determine the algae concentration in the water flow, and transmitting pool monitoring data representative of the algae concentration from the remote pool monitoring device to a mobile device or other user interface over a wireless network. In one example, the wirelessly transmitting step comprises transmitting the algae signal over a low-power wide-area network using a LoRa transceiver and the transmitting pool monitoring data step comprises transmitting the pool monitoring data over a Wi-Fi network.

At 304, the method includes an assessment process for comparing the algae concentration represented by the algae signal to a first threshold and a second threshold, outputting a first indication when the algae concentration is below the first threshold, outputting a second indication when the algae concentration is between the first threshold and the second threshold, and outputting a third indication when the algae concentration exceeds the second threshold, thereby enabling a pool owner or pool service technician to take appropriate remedial action in response to the detected algae concentration level. Optionally, the assessment process includes transmitting messages or otherwise notifying a pool owner of the remedial action to be taken.

The foregoing description is merely illustrative in nature and is in no way intended to limit the disclosure, its application, or uses. The broad teachings of the disclosure can be implemented in a variety of forms. Therefore, while this disclosure includes particular examples, the true scope of the disclosure should not be so limited since other modifications will become apparent upon a study of the drawings, the specification, and the following claims. It should be understood that one or more steps within a method may be executed in a different order (or concurrently) without altering the principles of the present disclosure. Further, although each of the embodiments is described above as having certain features, any one or more of those features described with respect to any embodiment of the disclosure can be implemented in and/or combined with features of any of the other embodiments, even if that combination is not explicitly described. In other words, the described embodiments are not mutually exclusive, and permutations of one or more embodiments with one another remain within the scope of this disclosure.

Spatial and functional relationships between elements (for example, between modules, circuit elements, semiconductor layers, etc.) are described using various terms, including "connected," "engaged," "coupled," "adjacent," "next to," "on top of," "above," "below," and "disposed." Unless explicitly described as being "direct," when a relationship between first and second elements is described in the above disclosure, that relationship can be a direct relationship where no other intervening elements are present between the first and second elements, but can also be an indirect relationship where one or more intervening elements are present (either spatially or functionally) between the first and second elements. As used herein, the phrase at least one of A, B, and C should be construed to mean a logical (A OR B OR C), using a non-exclusive logical OR, and should not be construed to mean "at least one of A, at least one of B, and at least one of C."

In the figures, the direction of an arrow, as indicated by the arrowhead, generally demonstrates the flow of information (such as data or instructions) that is of interest to the illustration. For example, when element A and element B exchange a variety of information but information transmitted from element A to element B is relevant to the illustration, the arrow may point from element A to element B. This unidirectional arrow does not imply that no other information is transmitted from element B to element A. Further, for information sent from element A to element B, element B may send requests for, or receipt acknowledgments of, the information to element A.

In this application, including the definitions below, the term "module" or the term "controller" may be replaced with the term "circuit." The term "module" may refer to, be part of, or include: an Application Specific Integrated Circuit (ASIC); a digital, analog, or mixed analog/digital discrete circuit; a digital, analog, or mixed analog/digital integrated circuit; a combinational logic circuit; a field programmable gate array (FPGA); a processor circuit (shared, dedicated, or group) that executes code; a memory circuit (shared, dedicated, or group) that stores code executed by the processor circuit; other suitable hardware components that provide the described functionality; or a combination of some or all of the above, such as in a system-on-chip.

The module may include one or more interface circuits. In some examples, the interface circuits may include wired or wireless interfaces that are connected to a local area network (LAN), the Internet, a wide area network (WAN), or combinations thereof. The functionality of any given module of the present disclosure may be distributed among multiple modules that are connected via interface circuits. For example, multiple modules may allow load balancing. In a further example, a server (also known as a remote or cloud) module may accomplish some functionality on behalf of a client module.

The term code, as used above, may include software, firmware, and/or microcode, and may refer to programs, routines, functions, classes, data structures, and/or objects. The term shared processor circuit encompasses a single processor circuit that executes some or all code from multiple modules. The term group processor circuit encompasses a processor circuit that, in combination with additional processor circuits, executes some or all code from one or more modules. References to multiple processor circuits encompass multiple processor circuits on discrete dies, multiple processor circuits on a single die, multiple cores of a single processor circuit, multiple threads of a single processor circuit, or a combination of the above. The term shared memory circuit encompasses a single memory circuit that stores some or all code from multiple modules. The term group memory circuit encompasses a memory circuit that, in combination with additional memories, stores some or all code from one or more modules.

The term memory circuit is a subset of the term computer-readable medium. The term computer-readable medium, as used herein, does not encompass transitory electrical or electromagnetic signals propagating through a medium (such as on a carrier wave); the term computer-readable medium may therefore be considered tangible and non-transitory. Non-limiting examples of a non-transitory, tangible computer-readable medium are nonvolatile memory circuits (such as a flash memory circuit, an erasable programmable read-only memory circuit, or a mask read-only memory circuit), volatile memory circuits (such as a static random access memory circuit or a dynamic random access memory circuit), magnetic storage media (such as an analog or digital magnetic tape or a hard disk drive), and optical storage media (such as a CD, a DVD, or a Blu-ray Disc).

The apparatuses and methods described in this application may be partially or fully implemented by a special-purpose computer created by configuring a general-purpose computer to execute one or more particular functions embodied in computer programs. The functional blocks, flowchart components, and other elements described above serve as software specifications, which can be translated into the computer programs by the routine work of a skilled technician or programmer.

The computer programs include processor-executable instructions that are stored on at least one non-transitory, tangible computer-readable medium. The computer programs may also include or rely on stored data. The computer programs may encompass a basic input/output system (BIOS) that interacts with hardware of the special-purpose computer, device drivers that interact with particular devices of the special-purpose computer, one or more operating systems, user applications, background services, background applications, etc.

The computer programs may include: (i) descriptive text to be parsed, such as HTML (hypertext markup language), XML (extensible markup language), or JSON (JavaScript Object Notation), (ii) assembly code, (iii) object code generated from source code by a compiler, (iv) source code for execution by an interpreter, (v) source code for compilation and execution by a just-in-time compiler, etc. As examples only, source code may be written using syntax from languages including C, C++, C#, Objective-C, Swift, Haskell, Go, SQL, R, Lisp, Java^{®}, Fortran, Perl, Pascal, Curl, OCaml, Javascript^{®}, HTML5 (Hypertext Markup Language 5th revision), Ada, ASP (Active Server Pages), PHP (PHP: Hypertext Preprocessor), Scala, Eiffel, Smalltalk, Erlang, Ruby, Flash^{®}, Visual Basic^{®}, Lua, MATLAB, SIMULINK, and Python^{®}.

## Claims

1. An algae detector, comprising:
a flow cell disposed within a fluid pathway of a cartridge configured to pass water through the flow cell for algae detection;
a light source configured to emit excitation light through the flow cell into the water at a predetermined wavelength selected to cause algae within the water to fluoresce;
a photosensor configured to detect an amount of fluorescent light emitted by the algae in response to the excitation light; and
an enclosure configured to house the flow cell, the cartridge, the light source, and the photosensor.

2. An offline loop algae detection system, comprising:
a first connector configured to connect to a first portion of a pool recirculation system used to recirculate water for a recreational body of water;
a second connector configured to connect to a second portion of the pool recirculation system having a pressure differential differing from that of the first portion; and
the algae detector of claim 1, wherein the cartridge includes an inlet port configured to fluidly connect an inlet of the fluid pathway to the first connector and an outlet port configured to fluidly connect an outlet of the fluid pathway to the second connector.

3. An algae detection and pool cleaning system, comprising:
a brush cleaner configured to clean a surface of a swimming pool containing a recreational body of water;
a water pump attached to the brush cleaner and configured to pump water from the recreational body through a water pump outlet; and
the algae detector of claim 1, wherein the cartridge includes an inlet port configured to fluidly connect an inlet of the fluid pathway to the water pump outlet.

4. An algae detection and pool cleaning system, comprising:
an automatic cleaner configured to automatically clean a surface of a swimming pool containing a recreational body of water using a motorized cleaning element;
a water pump attached to the automatic cleaner and configured to pump water from the recreational body through a water pump outlet; and
the algae detector of claim 1, wherein the cartridge includes an inlet port configured to fluidly connect an inlet of the fluid pathway to the water pump outlet.

5. The algae detection and pool cleaning system according to claim 4, wherein the automatic cleaner is one of a robotic cleaner, a suction side cleaner, and a pressure side cleaner.

6. An algae detection and pool cleaning system, comprising:
a self-propelled surface skimmer configured to clean proximate a waterline surface of a recreational body of water;
a water pump attached to the self-propelled surface skimmer and configured to pump water from the recreational body through a water pump outlet; and
the algae detector of claim 1, wherein the cartridge includes an inlet port configured to fluidly connect an inlet of the fluid pathway to the water pump outlet.

7. An algae detection and pool cleaning system, comprising:
a floating surface skimmer configured to clean proximate a waterline surface of a recreational body of water;
a water pump attached to the floating surface skimmer and configured to pump water from the recreational body through a water pump outlet; and
the algae detector of claim 1, wherein the cartridge includes an inlet port configured to fluidly connect an inlet of the fluid pathway to the water pump outlet.

8. A remote pool monitoring system, comprising:
a pool monitoring device; and
the algae detector according to claim 1, further comprising a wireless transceiver configured to wirelessly transmit an algae signal to the pool monitoring device, wherein the algae signal represents the amount of fluorescent light detected by the photosensor.

9. The remote pool monitoring system according to claim 8, wherein the wireless transceiver is a LoRa transceiver configured to transmit the algae signal over a low-power wide-area network to the pool monitoring device, wherein the pool monitoring device includes a Wi-Fi transceiver configured to transmit pool monitoring data over a Wi-Fi network.

10. The algae detector according to claim 1, wherein the flow cell includes an emitter side facing the light source and a sensor side facing the photosensor, wherein the emitter side is orthogonal to the sensor side.

11. The algae detector according to claim 1, wherein the cartridge includes an inlet port connected proximate to an inlet to the fluid pathway and an outlet port connected proximate to an outlet of the fluid pathway.

12. The algae detector according to claim 11, wherein the enclosure, the cartridge, the inlet port, and the outlet port cooperate to provide a light-tight fit configured to prevent ambient light from reaching the flow cell, the light source, and the photosensor.

13. The algae detector according to claim 11, wherein the inlet port and the outlet port are threadably connected to the cartridge and include raised annular rings configured to secure a flexible hose or tubing.

14. The algae detector according to claim 1, wherein the photosensor is included as part of a sensor assembly having an indicator configured to output an indication of the amount of the fluorescent light detected by the photosensor.

15. The algae detector according to claim 14, wherein the indicator is a visual indicator comprising one or more light emitting diodes visible from an exterior of the enclosure via a light pipe extending from the sensor assembly through a lid of the enclosure, wherein in particular
the visual indicator is configured to output a first indication when the amount of algae is below a first algae concentration threshold, a second indication when the amount of algae is between the first algae concentration threshold and a second algae concentration threshold, and a third indication when the amount of algae exceeds the second algae concentration threshold.
